(19) Europäisches Patentamt
European Patent Office
Office européen des brevets

(11) Veröffentlichungsnummer: **0 297 296 B1**

(12) **EUROPÄISCHE PATENTSCHRIFT**

(45) Veröffentlichungstag der Patentschrift: **17.02.93**

(51) Int. Cl.5: **C07C 217/42**

(21) Anmeldenummer: **88108660.7**

(22) Anmeldetag: **31.05.88**

(54) **Verfahren zur Herstellung von 2,2'-Oxybis[N,N-dimethyl-ethanamin].**

(30) Priorität: **30.06.87 DE 3721538**

(43) Veröffentlichungstag der Anmeldung:
**04.01.89 Patentblatt 89/01**

(45) Bekanntmachung des Hinweises auf die Patenterteilung:
**17.02.93 Patentblatt 93/07**

(84) Benannte Vertragsstaaten:
**BE DE FR GB IT NL**

(56) Entgegenhaltungen:
**EP-A- 0 006 454**
**EP-A- 0 115 071**

(73) Patentinhaber: **HOECHST AKTIENGESELLSCHAFT**
**Postfach 80 03 20**
**W-6230 Frankfurt am Main 80(DE)**

(72) Erfinder: **Weber, Jürgen, Dr. Dipl.-Chem.**
**Bunsenstrasse 17**
**W-4200 Oberhausen 11(DE)**
Erfinder: **Kampmann, Detlef, Dr. Dipl.-Chem.**
**Rankenweg 3**
**W-4630 Bochum(DE)**
Erfinder: **Thönnessen, Franz, Dr. Dipl.-Chem.**
**Lützowstrasse 49**
**W-4200 Oberhausen 11(DE)**
Erfinder: **Kniep, Claus**
**Rosenstrasse 93**
**W-4200 Oberhausen 1(DE)**

EP 0 297 296 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 2,2′-Oxybis[N,N-dimethylethanamin].

Dieses Produkt - auch Bis-[2-(N,N-dimethylamino)-ethyl]-ether genannt - stellt eine technisch bedeutsame Verbindung dar, die als Polymerisations-, Hartungs- und Verschäumungskatalysator für die Herstellung von Kunststoffen auf Epoxid- und insbesondere Urethanbasis dient.

2,2′-Oxybis [N,N-dimethylethanamin] kann auf verschiedenen Wegen synthetisiert werden.

Nach Lehre der US-PS 42 47 482 behandelt man N,N-Dimethylethanolamin mit gasförmigem Schwefeltrioxid und erhält unter Wasserabspaltung 63 bis 68 % Bis-[2-(N,N-dimethylamino)-ethyl]-ether.

Die US-PS 34 00 157 beschreibt die Umsetzung von in Wasser gelöstem Di-(2-chloroethyl)-ether mit im Überschuß eingesetzten Dimethylamin. Der gewünschte Ether wird durch Zugabe wäßrigen Alkalis freigesetzt. Bezogen auf Di-(2-chloroethyl)-ether beträgt die Ausbeute 66 % Bis-[2-N,N-dimethylamino)-ethyl]-ether.

Gemäß der DE 28 24 908 Al setzt man Dimethylamin, Diethylenglykol und Wasserstoff als Ausgangsstoffe unter Druck und erhöhter Temperatur in flüssiger Phase an einem festangeordneten Kupferkatalysator um. Führt man die nicht umgesetzten Ausgangsstoffe und den bei der Reaktion gebildeten Dimethylaminoglykolether direkt in die Synthese zurück, so erhält man ein Gemisch mit 60 Gew.-% Tetramethyl-bis-(aminoethyl)-ether (Bis-[2-(N,N′-dimethylamino)-ethyl]-ether).

Die vorstehend beschriebenen Verfahren sind einerseits recht aufwendig und liefern andererseits das Wertprodukt in relativ niedriger Ausbeute.

Daher besteht ein Bedarf nach einem Verfahren, das sowohl einfach zu praktizieren ist als auch den gewünschten Ether (2,2′-Oxybis[N,N-dimethylethanamin]) in hoher Ausbeute zugänglich macht.

Diese Aufgaben werden gelost durch ein Verfahren zur Herstellung von 2,2′-Oxybis[N,N-dimethylethanamin] durch Umsetzung eines Amins, einer sauerstoffhaltigen Verbindung und Wasserstoff als Ausgangsstoffe unter Druck und erhöhter Temperatur in flüssiger Phase an einem festangeordneten Katalysator.

Es ist dadurch gekennzeichnet, daß als Amin 2,2′-Oxybisethanamin, als sauerstoffhaltige Verbindung Formaldehyd eingesetzt wird, der festangeordnete Katalysator ein Ni, Cu, Co, Mn, Fe, Rh, Pd und/oder Pt enthaltender Hydrierkatalysator ist und die Ausgangsstoffe, insbesondere der Formaldehyd, einen verminderten Wasseranteil enthalten.

Die Ausgangsstoffe Formaldehyd und Wasserstoff sind großtechnisch erzeugte Substanzen. Das 2,2′-Oxybisethanamin (Bis-(2-Aminoethyl)-ether) ist gleichfalls leicht zugänglich, da es bei der Herstellung von Morpholin aus Ethanolamin stets als Nebenprodukt anfällt.

Die Ausgangsstoffe Bis-(2-aminoethyl)-ether, Formaldehyd und Wasserstoff werden bei 0,1 bis 30, insbesondere 1 bis 20, bevorzugt 2 bis 15 MPa umgesetzt. Die Reaktionstemperatur beträgt 20 bis 250, insbesondere 50 bis 200, bevorzugt 70 bis 150 ° C.

Das erfindungsgemäße Verfahren kann diskontinuierlich und kontinuierlich betrieben werden.

Die Reaktion folgt der Gleichung:

$$(H_2N-CH_2-CH_2)_2O + 4\ CH_2O + 4H_2 \longrightarrow ((CH_3)_2N-CH_2-CH_2)_2O + 4H_2O$$

Eine derartige Umsetzung bezeichnet man auch als hydrierende N-Methylierung des Amins. Eine zusammenfassende Übersicht bezüglich dieses Verfahrenstyps findet sich in Houben-Weyl, Methoden der organischen Chemie; Band XI/1, Seiten 641 bis 647, 4. Auflage (1957).

Als festangeordneter Katalysator kommen übliche Hydrierkontakte, die Ni, Co, Cu, Mn, Fe, Rh, Pd und/oder Pt enthalten, in Betracht. Druck und Temperatur sind in gewissem Umfange auch von der Art des Katalysators abhangig und mussen entsprechend aufeinander abgestimmt werden.

Wesentlich für das Gelingen des erfindungsgemäßen Verfahrens ist, daß die Ausgangsstoffe insbesondere der Formaldehyd und somit auch das im Reaktor vorliegende Gemisch einen begrenzten Wassergehalt aufweist.

Zu hohe Wasseranteile sind unerwünscht, da sie den festangeordneten Katalysator schädigen. Einerseits beeinträchtigen sie die Aktivität des Katalysators und verringern sowohl Umsatz als auch Selektivität der Reaktion, andererseits führen sie zu einem raschen Zerfall des festangeordneten Katalysators.

Eine Reduzierung des Wasseranteils in den Ausgangsstoffen trägt dazu bei, diesen Nachteil zu umgehen.

Das erfindungsgemäße Verfahren besitzt mehrere Vorteile: Es geht erstens von leicht zugänglichen Produkten aus und ist zweitens unproblematisch durchzuführen. Es gestattet drittens die Verwendung üblicher Hydrierkatalysatoren, wodurch das Verfahren sich flexibel an die jeweiligen betrieblichen Gegebenheiten anpassen läßt, und es liefert viertens das gewünschte Wertprodukt nahezu ohne Bildung von Nebenprodukten. So beträgt der Umsatz 99 bis 100 % und die Selektivität 96 bis 99,5 % bezogen auf eingesetztes 2,2′-Oxybisethanamin. Das Reaktionsgemisch enthalt das 2,2′-Oxybis[N,N-dimethyl-ethanamin] entsprechend einer Ausbeute von 95 bis 99,5 %.

Eine generelle Übertragung diskontinuierlicher Versuche auf eine kontinuierliche Umsetzung von insbesondere 2,2′-Oxybisethanamin (Bis-(2-Aminoethyl)-ether) - im folgenden vereinfacht Amin genannt - mit Formaldehyd und Wasserstoff ist allerdings nicht möglich.

Hierfur sind verschiedene Grunde verantwortlich.

Bei der kontinuierlichen N-Methylierung verwendet man üblicherweise druckfeste Rohrreaktoren, die den Hydrierkatalysator in stückiger Form enthalten. Die Ausgangsstoffe oder deren Umsetzungsgemische werden dem Reaktor entweder am Kopf oder am Boden eingeführt. Je nach Zugabeart spricht man von Riesel- oder Sumpffahrweise. Bei längerem Gebrauch zerfallen die Hydrierkatalysatoren in zunehmendem Maße. Die resultierenden feinteiligen Partikel führen entweder in den Rohrreaktoren oder in nachgeschalteten Anlageteilen zu Verlegungen. Dadurch kommt es zu einem Druckanstieg in der Reaktionsapparatur, mit der zwangsweisen Folge, daß die Umsetzung unterbrochen werden muß, um die Verstopfungen zu beseitigen.

Ebenfalls unerwünscht ist die Entstehung von Ameisensäure, die sich vermutlich durch Cannizzaro-Reaktion aus Formaldehyd bildet. Sie entzieht der Reaktion eine entsprechende Menge Amin als Salz. Die freie Säure wie auch das Aminsalz begünstigen Korrosionen im Reaktorsystem.

Weitere, eine kontinuierliche Umsetzung storende Nebenreaktionen beruhen auf der Polymerisation von Formaldehyd und der Polykondensation zwischen dem Amin und Formaldehyd zu Hexahydrotriazinen oder im Falle mehrwertiger Amine zu höhermolekularen Verbindungen. Die Bildung polymerer Stoffe führt zu Verklebungen der Katalysatorfüllung und somit zu Blockierung des Reaktors. Ein Kontaktwechsel ist die unvermeidliche Folge.

Entscheidend für ein kontinuierlich durchgeführtes Verfahren ist ferner, daß die Ausgangsstoffe voneinander getrennt auf eine vorgegebene Temperatur erhitzt und anschließend in Gegenwart des festangeordneten Katalysators unter Vermischung zusammengeführt werden. Dies bedeutet, daß man Amin, Formaldehyd und Wasserstoff über eigene Leitungen führt und erwärmt, bevor man sie reagieren läßt.

Man kann auch den Wasserstoff im Gemisch mit Amin oder in Gemisch mit Formaldehyd unter Vorerwärmen der Reaktion zuleiten. Es ist auch möglich, den Wasserstoff auf Amin und Formaldehyd zu verteilen.

Nach einer besonderen Ausführungsform wird Wasserstoff mit Amin vermischt und erwärmt.

Hingegen ist es nicht zulässig, Amin und Formaldehyd zu mischen und zu erwärmen, ehe die Reaktion erfolgt. Amin und Formaldehyd sind stets voneinander getrennt zu führen und auf eine vorgegebene Temperatur aufzuheizen, bevor sie in Gegenwart des festangeordneten Katalysators zur Reaktion gelangen.

Beachtet man die Anweisung, die Ausgangsstoffe getrennt in die Katalysatorzone zu führen, nicht, sondern bringt sie vor Eintritt in die Katalysatorzone zur Vermischung, so ergeben sich vermutlich infolge von Polymerisationsreaktionen sehr bald Verbackungen und Verklebungen des festangeordneten Katalysators. Die Folge ist, daß der Druckreaktor infolge Verlegungen blockiert wird und die Umsetzung abgebrochen werden muß.

Ferner muß darauf geachtet werden, den Wassergehalt in der Reaktion zu begrenzen. Dies erreicht man, indem man Ausgangsstoffe mit vermindertem Wassergehalt verwendet. Das Amin weist Wasser nur in untergeordneten Mengen oder in Spuren auf. Dagegen sind in dem technisch gebräuchlichen Formaldehyd, der etwa 60 Gew.-% und mehr Wasser aufweisen kann, beträchtliche Wasseranteile enthalten. Zu diesem Wasser, das mit den Ausgangsstoffen in die Reaktion eingebracht wird, kommt noch das durch die Umsetzung freigesetzte Reaktionswasser hinzu.

Die Summe beider Wasseranteile soll, bezogen auf das den festangeordneten Katalysator umströmende Flüssigkeits-Gas-Gemisch einen Wert von 50 Gew.-% nicht übersteigen.

Besonders zweckmäßig ist es, den Wassergehalt der Formaldehydlösung zu begrenzen. Er soll einen Wert von 50 Gew.-% bezogen auf Formaldehydlösung nicht übersteigen. Geeignet sind Formaldehydlösungen mit 0 bis 50 Gew.-% Wasser. Besonders bewährt haben sich solche mit 5 bis 30 Gew.-% Wasser. Bevorzugt wird ein Formaldehyd mit 7 bis 15 Gew.-% Wasser. Mit gutem Erfolg kann auch Paraformaldehyd, z.B. in suspendierter Form eingesetzt werden.

Das Amin und/oder der Formaldehyd können in einem Solvens gelöst vorliegen. Geeignete Lösungsmittel sind sowohl aliphatische Alkohole mit 1 bis 5 Kohlenstoffatomen, Ether wie Tetrahydrofuran und Dioxan sowie Dimethylsulfoxid als auch Mischungen der vorstehend genannten Stoffe.

Besonders günstig ist es, den Formaldehyd als Lösung in einem der obengenannten Solventien zu verwenden.

Die vorgegebene Temperatur richtet sich im wesentlichen nach der Reaktionstemperatur. Sie sollte nicht mehr als 20, insbesondere 10, bevorzugt 5 ° C unterhalb der Reaktionstemperatur und nicht mehr als 10, insbesondere 5, bevorzugt 0 ° C oberhalb der Reaktionstemperatur liegen.

Empfehlenswert ist ein Bereich von 20 bis 0, insbesondere 10 bis 0, bevorzugt 5 bis 0 ° C unterhalb der Reaktionstemperatur.

Nachdem die Ausgangsstoffe auf die vorgegebene Temperatur erwärmt worden sind, führt man sie in Gegenwart des festangeordneten Katalysators zusammen. Alle drei Ausgangsstoffe Amin, Formaldehyd und Wasserstoff dürfen erst in Gegenwart des festangeordneten Katalysators aufeinandertreffen.

Wird der Wasserstoff zusammen mit einem der übrigen Ausgangsstoffe der Reaktion zugeleitet, so dürfen das Amin und der Formaldehyd erst in Anwesenheit des festangeordneten Katalysators zusammengeführt werden. Auch in diesem Falle geraten alle drei Ausgangsstoffe nur im Beisein des Kontaktes miteinander in Berührung. Technisch realisiert man diese Forderung dadurch, daß man die Leitung oder das Leitungssystem wenigstens eines der beiden Ausgangsstoffe Amin und Formaldhyd in der Katalysatorzone enden läßt. Somit wird sichergestellt, daß das Amin oder der Formaldehyd nur im Zugegensein von Katalysator austritt und mit den anderen Reaktionsteilnehmern zusammentrifft.

Nach einer bevorzugten Ausführungsform verläßt der Formaldehyd die in der Katalysatorzone endende Leitung.

Es ist jedoch auch möglich, die Leitungen zweier oder auch aller drei Ausgangsstoffe in der Zone des festangeordneten Katalysators münden zu lassen. Welche Anordnung der Leitungen zu wählen ist, wird zum einen von den Stoffmengen und zum anderen von der Geometrie der Kontaktzone und den erforderlichen Strömungsverhältnissen abhängen. Bei Durchsatz größerer Stoffmengen je Zeiteinheit empfiehlt es sich, je Ausgangsstoff nicht nur eine, sondern mehrere Leitungen zu gebrauchen. Sollten die Strömungsverhältnisse eine ausreichende Durchmischung der Reaktanten in der Katalysatorzone nicht sicherstellen, ist die Verwendung zusätzlicher Verteilervorrichtungen nützlich. Diese Verteiler können beispielsweise als Ringbrause oder Duschkopf am Ende einer Leitung angebracht werden. Aber auch andere Verteilersysteme wie Düsen, Fritten oder Röhrenbündel können Verwendung finden.

Das erfindungsgemäße Verfahren ist nicht auf den Gebrauch spezieller Katalysatoren beschränkt, sondern gestattet die Verwendung einer Vielzahl üblicher festangeordneter Katalysatoren.

Die festangeordneten Katalysatoren können Trägerstoffe besitzen, aber auch frei von Trägern sein. Sie enthalten Ni, Co, Cu, Mn, Fe, Rh, Pd und/oder Pt, bevorzugt Ni, Co und/oder Pd und daneben gegebenenfalls gebräuchliche Zusatzstoffe und Promotoren, beispielsweise Erdalkalioxide, $SiO_2$, $Al_2O_3$, $MnO_2$ und / oder $Cr_2O_3$.

Vorteilhaft ist der Gebrauch von Trägerkatalysatoren. Als Träger kommen $Al_2O_3$, $SiO_2$, Kieselgel, Kieselerde, Aktivkohle, Bimsstein in Betracht.

Besonders günstig ist die Verwendung von Katalysatoren, mit 10 bis 75 insbesondere 20 bis 70, bevorzugt 40 bis 65 Gew.-% Ni, Co, Cu, Mn und/oder Fe bezogen auf gesamte Katalysatormasse.

Edelmetallkatalysatoren gestatten eine Umsetzung unter besonders milden Bedingungen. Sie sind üblicherweise auf einem Träger untergebracht und weisen einen Metallgehalt von 0,1 bis 20 insbesondere 0,2 bis 15, bevorzugt 0,5 bis 10 Gew.-% bezogen auf gesamte Katalysatormasse auf. Geeignete Edelmetalle sind Rh, Pd und/oder Pt. Als Träger empfehlen sich geformte Materialien auf Basis von $Al_2O_3$, $SiO_2$, Aktivkohle, Kieselgel, Kieselgur und/oder Bimsstein.

Der Katalysator bestimmt die Reaktionsbedingungen, insbesondere die Reaktionstemperatur und den Druck.

Die Ni, Co, Cu, Mn und/oder Fe-haltigen Nichtedelmetallkatalysatoren erfordern 50 bis 250, insbesondere 70 bis 200, bevorzugt 100 bis 150 ° C und 3 bis 30, insbesondere 5 bis 20, bevorzugt 8 bis 15 MPa.

Edelmetallkatalysatoren benötigen 20 bis 165, insbesondere 30 bis 160, bevorzugt 50 bis 150 ° C und 0,1 bis 15, insbesondere 0,2 bis 12, bevorzugt 0,5 bis 10 MPa. Besonders schonende Bedingungen liefern Temperaturen bis 100 ° C. Eine schnellere Umsetzung erfolgt in einem Bereich von 100 bis 165, insbesondere 110 bis 160, bevorzugt 115 bis 150 ° C.

Je Mol Amin werden 4 bis 8, insbesondere 4 bis 6, bevorzugt 4 bis 4,8 Mol Formaldehyd eingesetzt. Je Mol Formaldehyd werden 1 bis 10 insbesondere 1,05 bis 5, bevorzugt 1,1 bis 2,5 Mol $H_2$ der Reaktion zugeleitet.

Experimenteller Teil:

Der Reaktorbehälter besteht aus einem druckfesten Rohr mit einem Innendurchmesser von 28 mm. In den unteren Teil des Druckrohres werden Raschigringe, die einen Durchmesser von 3 mm aufweisen, bis zu einer Höhe von 800 mm eingefüllt. Diese Zone dient als Vorheizzone, in der die Ausgangsstoffe auf die vorgegebene Temperatur aufgeheizt werden. Die Beheizung erfolgt über einen den Reaktor umgebenden Heizmantel. Oberhalb der Vorheizzone befindet sich der festange-rdnete Katalysator.

Der Reaktor wird zu Beginn der Umsetzung mit Bis-(aminoethyl)-ether befüllt. Die Ausgangsstoffe werden von unten in den Reaktor eingepumpt, das Reaktionsgemisch wird oberhalb der Katalysatorzone abgezogen und einem Druckabscheider zugeführt.

Das Amin wird zusammen mit Wasserstoff dem Reaktor zugeleitet und durchströmt die mit Raschigringen befüllte Vorheizzone, während der Formaldehyd über ein separates Steigrohr, das durch die Vorheizzone führt, direkt in die Schicht des festangeordneten Katalysators gepumpt und erst dort mit dem Amin und Wasserstoff vermischt wird. Die Vorheizzone wird mittels des den Reaktor außen umgebenden Heizmantels erhitzt und die Ausgangsstoffe werden auf die gewünschte vorgegebene Temperatur gebracht.

Beispiel 1:

Die Umsetzung wird 860 Stunden unter den in der nachfolgenden Tabelle aufgeführten Bedingungen kontinuierlich durchgeführt. Es treten keinerlei Komplikationen auf. Nach Beendigung der Umsetzung wird der Katalysator dem Reaktor zur Überprüfung entnommen. Er weist weder Zerfallserscheinungen noch Verklebungen auf und ist unvermindert aktiv.

Beispiel 1a:

Beispiel 1 wird wiederholt, davon abweichend jedoch werden Amin und Formaldehyd in der Vorheizzone zusammengeführt und als Mischung der Katalysatorzone zugeleitet. Bereits nach 20 Stunden tritt ein starker Leistungsabfall des Katalysators auf. Nach insgesamt 50 Stunden muß die Reaktion wegen Verlegung des Druckreaktors (Bildung von Polymerisaten) abgebrochen werden.

Beispiel 2:

Die Umsetzung wird 120 Stunden unter den in der nachfolgenden Tabelle aufgeführten Bedingungen kontinuierlich durchgeführt. Es treten keinerlei Komplikationen auf. Nach Beendigung der Umsetzung wird der Katalysator dem Reaktor zur Überprüfung entnommen. Er weist weder Zerfallserscheinungen noch Verklebungen auf und war unvermindert aktiv.

Beispiel 2a:

Beispiel 2 wird wiederholt, davon abweichend jedoch werden Amin und Formaldehyd in der Vorheizzone zusammengeführt und als Mischung der Katalysatorzone zugeleitet. Bereits nach 35 Stunden tritt ein starker Leistungsabfall des Katalysators auf. Nach insgesamt 90 Stunden muß die Reaktion wegen Verlegung des Druckreaktors (Bildung von Polymerisaten) abgebrochen werden.

Die Reaktionsbedingungen und die Reaktionsergebnisse (Beispiel 1 und 2) sind in der nachfolgenden Tabelle zusammengefaßt.

Tabelle

| | Beispiel 1 | Beispiel 2 |
|---|---|---|
| **Reaktionsbedingungen:** | | |
| Vorgegebene Temperatur | 146 bis 147°C | 125 bis 127°C |
| Reaktionstemperatur | 150°C | 130°C |
| Reaktionsdruck | 10 MPa | 10 MPa |
| Katalysator | 230 ml Ni 52/35* | 200 ml Pd/$Al_2O_3$** |
| **Einsatz:** | | |
| Bis-(aminoethyl)-ether | 35 ml/h | 30 ml/h |
| Formaldehyd*** | 65 bis 70 ml/h | 60 bis 65 ml/h |
| **Zusammensetzung des Reaktionsgemisches:** | | |
| Bis-[2-(N,N-dimethylamino)-ethyl]-ether | 51,2 Gew.-% | 50,7 Gew.-% |
| Wasser | 26,5 Gew.-% | 27,2 Gew.-% |
| Methanol | 20,1 Gew.-% | 20,4 Gew.-% |
| Sonstige | 2,2 Gew.-% | 1,7 Gew.-% |

* nickelhaltiger Katalysator mit 48 bis 53 Gew.-% Ni bezogen auf Katalysatormasse, Trägermaterial Kieselgur, Handelsprodukt der Hoechst AG

** Palladium-Katalysator: 10 Gew.-% Pd bezogen auf Katalysatormasse, Trägermaterial $Al_2O_3$

*** Formaldehydlösung (55 Gew.-% Formaldehyd, 35 Gew.-% Methanol, 10 Gew.-% Wasser)

## Patentansprüche

**1.** Verfahren zur Herstellung von 2,2'-Oxybis[N,N-dimethylethanamin] durch Umsetzung eines Amins, einer sauerstoffhaltigen Verbindung und Wasserstoff als Ausgangsstoffe unter erhöhter Temperatur und Druck an einem festangeordneten Katalysator, dadurch gekennzeichnet, daß als Amin 2,2'-Oxybisethanamin, als sauerstoffhaltige Verbindung Formaldehyd eingesetzt werden, der festangeordnete Katalysator ein Ni, Co, Cu, Mn, Fe, Rh, Pd und/oder Pt enthaltender Hydrierkatalysator ist und die Ausgangsstoffe, insbesondere der Formaldehyd einen verminderten Wasseranteil enthalten.

2. Verfahren nach Anspruch 1, dadurch gekennzeichnet, daß der Druck 0,1 bis 30, insbesondere 1 bis 20, bevorzugt 2 bis 15 MPa und die Temperatur 20 bis 250, insbesondere 50 bis 200, bevorzugt 70 bis 150 ° C beträgt.

3. Verfahren nach Anspruch 1 oder 2, dadurch gekennzeichnet, daß die Ausgangsstoffe voneinander getrennt auf eine vorgegebene Temperatur erhitzt und anschließend in Gegenwart des festangeordneten Katalysators unter Vermischung zusammengeführt werden.

4. Verfahren nach einem oder mehreren der Ansprüche 1 bis 3, dadurch gekennzeichnet, daß Amin und Formaldehyd voneinander getrennt auf die vorgegebene Temperatur erhitzt werden und anschließend in Gegenwart des festangeordneten Katalysators zusammengeführt werden.

5. Verfahren nach einem oder mehreren der Ansprüche 1 bis 4, dadurch gekennzeichnet, daß der Formaldehyd in gelöster Form eingesetzt wird und die Formaldehydlösung 0 bis 50, insbesondere 5 bis 30, bevorzugt 7 bis 15 Gew.-% Wasser enthält.

6. Verfahren nach einem oder mehreren der Ansprüche 3 bis 5, dadurch gekennzeichnet, daß die vorgegebene Temperatur nicht mehr als 20, insbesondere 10 bevorzugt 5 ° C unterhalb der Reaktionstemperatur und nicht mehr als 10, insbesondere 5, bevorzugt 0 ° C oberhalb der Reaktionstemperatur liegt.

7. Verfahren nach einem oder mehreren der Ansprüche 3 bis 6, dadurch gekennzeichnet, daß die vorgegebene Temperatur 20 bis 0, insbesondere 10 bis 0, bevorzugt 5 bis 0 ° C unterhalb der Reaktionstemperatur liegt.

8. Verfahren nach einem oder mehreren der Ansprüche 1 bis 7, dadurch gekennzeichnet, daß der Katalysator ein Trägerkatalysator oder ein trägerfreier Metallkatalysator ist.

9. Verfahren nach einem oder mehreren der Ansprüche 1 bis 8, dadurch gekennzeichnet, daß der Katalysator Ni, Co, Cu, Mn und/oder Fe und daneben gegebenenfalls gebräuchliche Zusatzstoffe und Promotoren enthält.

10. Verfahren nach einem oder mehreren der Ansprüche 1 bis 9, dadurch gekennzeichnet, daß der Katalysator Rh, Pd und/oder Pt und daneben gegebenenfalls gebräuchliche Zusatzstoffe und Promotoren enthält.

11. Verfahren nach einem oder mehreren der Ansprüche 1 bis 10, dadurch gekennzeichnet, daß der Katalysator als Träger $Al_2O_3$, $SiO_2$, Kieselgel, Kieselerde, Aktivkohle und/oder Bimsstein enthalt.

12. Verfahren nach einem oder mehreren der Ansprüche 1 bis 11, dadurch gekennzeichnet, daß als Zusatzstoffe und Promotoren im Katalysator Erdalkalioxide, $SiO_2$, $Al_2O_3$, $MnO_2$ und/oder $Cr_2O_3$ enthalten sind.

**Claims**

1. A process for the preparation of 2,2'-oxybis[N,N-dimethylethanamine] by the reaction of an amine, an oxygen-containing compound and hydrogen as starting materials at elevated temperature and pressure on a fixed-bed catalyst, characterised in that 2,2'-oxybisethanamine is used as the amine, formaldehyde as the oxygen-containing compound, the fixed-bed catalyst is a Ni, Co, Cu, Mn, Fe, Rh, Pd and/or Pt-containing hydrogenation catalyst and the starting materials, in particular the formaldehyde, contain a reduced percentage of water.

2. A process according to claim 1, characterised in that the pressure is 0.1 to 30, in particular 1 to 20 and preferably 2 to 15 MPa and the temperature is 20 to 250, in particular 50 to 200 and preferably 70 to 150 ° C.

3. A process according to claim 1 or 2, characterised in that the starting materials are heated to a preset temperature separate from each other and then mixed together in the presence of a fixed-bed catalyst.

4. A process according to one or several of the claims 1 to 3, characterised in that the amine and formaldehyde are heated to the preset temperature separate from each other and then added to each other in the presence of the fixed-bed catalyst.

5. A process according to one or several of the claims 1 to 4, characterised in that the formaldehyde is added in dissolved form and the formaldehyde solution contains 0 to 50, in particular 5 to 30 and preferably 7 to 15 wt.% water.

6. A process according to one or several of the claims 3 to 5, characterised in that the preset temperature is not more than 20 ° C, in particular 10 ° C and preferably 5 ° C below the reaction temperature and not more than 10 ° C, in particular 5 ° C and preferably 0 ° C above the reaction temperature.

7. A process according to one or several of the claims 3 to 6, characterised in that the preset temperature is 20 to 0, in particular 10 to 0 and preferably 5 to 0 ° C below the reaction temperature.

8. A process according to one or several of the claims 1 to 7, characterised in that the catalyst is a supported catalyst or a support-free metal catalyst.

9. A process according to one or several of the claims 1 to 8, characterised in that the catalyst contains Ni, Co, Cu, Mn and/or Fe and in addition in some cases conventional additives and promotors.

10. A process according to one or several of the claims 1 to 9, characterised in that the catalyst contains Rh, Pd and/or Pt and in addition in some cases conventional additives and promotors.

11. A process according to one or several of the claims 1 to 10, characterised in that the catalyst contains $Al_2O_3$, $SiO_2$, silica gel, kieselguhr, activated carbon and/or pumice as a support.

12. A process according to one or several of the claims 1 to 11, characterised in that the catalyst contains alkaline earth oxide, $SiO_2$, $Al_2O_3$, $MnO_2$ and/or $Cr_2O_3$ as additives and promotors.

**Revendications**

1. Procédé pour la fabrication de 2,2'-oxybis[N,N-diméthyléthanamine] par réaction d'une amine, d'un composé oxygéné et d'hydrogène comme matières premières à température et sous pression élevées sur un catalyseur stationnaire, caractérisé en ce que l'on utilise comme amine la 2,2'-oxybiséthanamine, comme composé oxygéné de formaldéhyde, le catalyseur stationnaire est un catalyseur d'hydrogénation contenant Ni, Co, Cu, Mn, Fe, Rh, Pd et/ou Pt et les matières premières, en particulier le formaldéhyde, contiennent une proportion d'eau réduite.

2. Procédé selon la revendication 1, caractérisé en ce que la pression est de 0,1 à 30 MPa, en particulier de 1 à 20 MPa, de préférence de 2 à 15 MPa et la température est de 20 à 250 ° C, en particulier de 50 à 200 ° C, de préférence 70 à 150 ° C.

3. Procédé selon la revendication 1 ou 2, caractérisé en ce que les matières premières sont chauffées séparément l'une de l'autre à une température prédéfinie et ensuite réunies et mélangées en présence du catalyseur stationnaire.

4. Procédé selon une ou plusieurs des revendications 1 à 3, caractérisé en ce que l'amine et le formaldéhyde sont chauffés séparément l'un de l'autre à la température prédéfinie et ensuite réunis en présence du catalyseur stationnaire.

5. Procédé selon une ou plusieurs des revendications 1 à 4, caractérisé en ce que le formaldéhyde est utilisé sous forme dissoute et la solution de formaldéhyde contient 0 à 50 % en poids d'eau, en particulier 5 à 30 % en poids, de préférence 7 à 15 % en poids.

6. Procédé selon une ou plusieurs des revendications 3 à 5, caractérisé en ce que la température prédéfinie est de pas plus de 20 ° C, en particulier 10 ° C, de préférence 5 ° C au-dessous de la température de réaction et pas plus de 10 ° C en particulier 5 ° C, de préférence 0 ° C au-dessus de la

température de réaction.

**7.** Procédé selon une ou plusieurs des revendications 3 à 6, caractérisé en ce que la température prédéfinie est de 20 à 0°C, en particulier 10 à 0°C, de préférence 5 à 0°C au-dessous de la température de réaction.

**8.** Procédé selon une ou plusieurs des revendications 1 à 7, caractérisé en ce que le catalyseur est un catalyseur sur support ou un catalyseur métallique sans support.

**9.** Procédé selon une ou plusieurs des revendications 1 à 8, caractérisé en ce que le catalyseur contient Ni, Co, Cu, Mn et/ou Fe et en outre éventuellement des additifs et promoteurs habituels.

**10.** Procédé selon une ou plusieurs des revendications 1 à 9, caractérisé en ce que le catalyseur contient Rh, Pd et/ou Pt et en outre éventuellement des additifs et promoteurs habituels.

**11.** Procédé selon une ou plusieurs des revendications 1 à 10, caractérisé en ce que le catalyseur contient comme support $Al_2O_3$, $SiO_2$, du gel de silice, de la silice, du charbon actif et/ou de la pierre ponce.

**12.** Procédé selon une ou plusieurs des revendications 1 à 11, caractérisé en ce que le catalyseur contient comme additifs et promoteurs des oxydes alcalino-terreux, $SiO_2$, $Al_2O_3$, $MnO_2$ et/ou $Cr_2O_3$.